# EUROPEAN PATENT APPLICATION

(11) **EP 4 306 086 A1**
(43) Date of publication of application: **17.01.2024**
(21) Application number: 22766142.8
(22) Date of filing: 22.02.2022
(51) Int. Cl.: A61F 5/00

(54) **SELF-CLOSING SYSTEM**

(30) Priority: 09.03.2021 CN 202110254649; 09.03.2021 CN 202120504661 U
(71) Applicant: Changzhou Panda Medical Co., Ltd., Changzhou, Jiangsu 213149 (CN)
(72) Inventor: SHI, Xiufeng, Shanghai 201318 (CN); ZHANG, Qiang, Shanghai 201318 (CN); LU, Lan, Shanghai 201318 (CN)
(74) Representative: Plasseraud IP
(86) International application number: PCT/CN2022/077156
(87) International publication number: WO 2022/188616

(57) **Abstract**

The present disclosure provides a self-sealing system of a balloon system implanted in vivo. The balloon system includes a balloon, a catheter and a filling; the balloon includes a self-sealing valve, which penetrates through the catheter and is hermetically connected to the catheter; a distal end of the self-sealing valve is provided with an inverted pocket and a sealing belt, the catheter penetrates through a small hole in the self-sealing valve and enters the inverted pocket, and the balloon is packaged in a capsule before being swallowed; and one end of the catheter is connected to the self-sealing valve on the balloon while the other end is left outside the capsule, and the catheter has a certain length, making an in-vitro end leave outside an oral cavity during use. The sealing belt may make the distal end of the inverted pocket be tightly closed after the filling is full and the catheter is removed, in order to prevent the filling from leaking out.

## Description

### TECHNICAL FIELD

The present disclosure relates to a balloon system implanted in vivo, in particular to a self-sealing system of the balloon system implanted in vivo.

### BACKGROUND

Over the past 20 years, the morbidity of the obesity worldwide, including the adult obesity, the elderly obesity and the adolescent and child obesity, has soared. According to the statistics of the World Health Organization, 15% of adult women and 11 % of adult men all over the world were obese in 2014, meaning that near one billion adults over the world were obese in 2014. What is not optimistic is that China has overtaken the United States in obesity. China had 89.6 million obese populations (the United States had 87.8 million obese populations) in 2016, including 43.2 million male populations and 46.4 million female populations. Obesity is often accompanied by hypertension, type II diabetes, ischemic heart disease, dyslipidemia, joint degeneration, sleep apnea, some tumors and the like, and therefore how to control body weight effectively, prevent and control the occurrence of complications is a topic of great concern.

At present, the main treatments for the obesity include diet control, life intervention, drug treatment and surgical treatment. However, the simple diet control, life intervention and drug treatment are hard to achieve the satisfactory weight-loss effect; and although the bariatric surgery can reach an obvious effect, it is hard for a patient to accept due to culture, medical level, surgical complications and other reasons. By simulating the mechanism of treating obesity through the surgical operation, including an intragastric balloon, implantable medical device weight loss just rises in these years and has attracted wide attention due to its safety, simplicity and convenience, efficiency and recovery, and it will become the main effective means for weight loss.

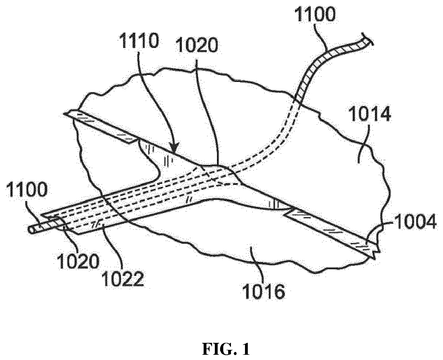

At present, several kinds of intragastric balloons have been listed abroad, particularly the intragastric balloon that is swallowed without requiring gastroscope delivery, so it is the direction of the weight-loss means in the future. Before taking effect, the swallowing intragastric balloon is packaged in a capsule and connected to a longer catheter, and one end of the catheter is connected to a self-sealing valve on the intragastric balloon while the other end is left outside the capsule and the oral cavity. The balloon, packaged in the capsule, and partial catheter are fed to a patient's stomach in a swallowing manner, the capsule degrades rapidly in a gastric acid environment, a filling is conveyed to the inside of the balloon from an in-vitro end of the catheter left outside the patient's oral cavity, and the balloon expands into an expanded state from an initial compressed state. After the conveying of the filling is finished, the catheter is removed from the patient in vivo, and the self-sealing valve, forming a fluid channel with the catheter, on the balloon, is closed automatically, in order to prevent the filling from leaking out. US patent No.US8974483B2 discloses such self-sealing valve with a catheter and a balloon, specifically as shown in FIG. 1:

The self-sealing valve with the catheter and the balloon disclosed by the US patent No.US8974483B2 includes a catheter 1100 and a two-layer self-sealing valve 1110 which is sealed along an edge. The self-sealing valve 1110 may be located in a hole on a material surface of an upper part 1014 or a lower part 1016 of the balloon, or in an opening of a seam 1004 of the upper part 1014 and the lower part 1016. The catheter 1100 enters the balloon through a channel 1020 between the two-layer self-sealing valve 1110 from the outside of the balloon, the catheter 1100 is removed after conveying the filling material in the balloon, and the channel 1020 between the two-layer self-sealing valve 1110 is sealed automatically, to prevent the filling in the balloon from leaking out.

The defect of such structure is that the channel 1020 between the two-layer self-sealing valve 1110 completely depends on self-sealing, and after long-term placement, the catheter 1100 will cause the channel 1020 to deform. Therefore, the deformed channel 1020 cannot be closed completely after the balloon filling is finished and the catheter 1100 is removed, and the filling in the balloon will leak out.

### SUMMARY

The present disclosure provides a seal-sealing system, making a catheter have a better sealing effect after being removed from a balloon, in order to prevent a filling from leaking.

An intragastric balloon system in The present disclosure includes a balloon, a catheter and a filling. A self-sealing valve may be located at any position of the balloon, the seal-sealing valve may be passed through the catheter and hermetically connected to the catheter, and a distal end of the self-sealing valve is provided with an inverted pocket and a sealing belt.

Preferably, the catheter passes through a small hole on the the self-sealing valve and enters the inverted pocket.

Preferably, the sealing belt is connected to the balloon.

Preferably, the balloon is packaged in a capsule before being swallowed, one end of the catheter is connected to the self-sealing valve on the balloon while the other end is left outside the capsule, and the catheter has a certain length, making an in-vitro end leave outside an oral cavity during use.

Preferably, the balloon includes an upper-layer main body and a lower-layer main body, and the self-sealing valve includes an upper-layer self-sealing valve and a lower-layer self-sealing valve.

Preferably, a small hole is formed in the lower-layer self-sealing valve, and a perimeter of the small hole is not greater than an outer diameter of the catheter.

Preferably, the upper-layer seal-sealing valve and the upper-layer main body are a whole, or connected together through different materials in a bonding manner or in a welding manner. The lower-layer seal-sealing valve and the lower-layer main body are a whole, or connected together through different materials in a bonding manner or in a welding manner.

Preferably, the inverted pocket includes an upper-layer inverted pocket membrane and a lower-layer inverted pocket membrane.

Preferably, the upper-layer inverted pocket membrane is an extension of the lower-layer self-sealing valve, or the upper-layer inverted pocket membrane and the lower-layer self-sealing valve are connected together through different materials in a bonding manner or in a welding manner.

Preferably, a length of the lower-layer inverted pocket membrane is greater than that of the upper-layer inverted pocket membrane.

Preferably, while the upper-layer main body and the lower-layer main body are combined together at the edge in a bonding manner or in a welding manner, three edges of the upper-layer self-sealing valve and the lower-layer self-sealing valve are bonded or welded together through a connecting belt, in order to form the self-sealing valve.

Preferably, all or a part of the lower-layer self-sealing valve and the upper-layer inverted pocket membrane are bonded or welded with the lower-layer inverted pocket membrane through the connecting belt, in order to form the inverted pocket; and the catheter is threaded in from unconnected ends of the upper-layer self-sealing valve and the lower-layer self-sealing valve and threaded out from the small hole, to enter any position of the inverted pocket.

Preferably, the sealing belt is at the outside of the inverted pocket.

Preferably, the sealing belt includes an upper-layer sealing strip and a lower-layer sealing strip, where the upper-layer sealing strip is at the outside of the upper-layer inverted pocket membrane, and the lower-layer sealing strip is at the outside of the lower-layer inverted pocket membrane.

Preferably, a length of the upper-layer sealing strip is not less than that of the lower-layer sealing strip, and a length of the lower-layer sealing strip is greater than a width of the lower-layer inverted pocket membrane.

Preferably, two sides of the lower-layer sealing strip are directly or indirectly connected to the upper-layer sealing strip through the inverted pocket in a bonding manner or a welding manner, in order to form the sealing belt.

Preferably, the sealing belt is adhered to the balloon through two ends of the upper-layer sealing strip.

Preferably, a length of the lower-layer sealing strip is not less than that of the upper-layer sealing strip, and a length of the upper-layer sealing strip is greater than a width of the upper-layer inverted pocket membrane.

Preferably, two sides of the upper-layer sealing strip are directly or indirectly connected to the lower-layer sealing strip through the inverted pocket in a bonding manner or a welding manner, in order to form the sealing belt.

Preferably, the sealing belt is adhered to the balloon through two ends of the lower-layer sealing strip.

Preferably, overturn is performed through the hole in the upper-layer main body after the combination is completed, the upper-layer main body and the lower-layer main body form the balloon, the self-sealing valve enters the inside of the balloon together with the inverted pocket and the sealing belt that are at the distal end of the self-sealing valve, one end of the catheter is outside the balloon while the other end is inside the inverted pocket, and finally the hole in the upper-layer main body is sealed with a patch.

Preferably, the small hole is formed in a pocket membrane of the inverted pocket, and the perimeter of the small hole is not greater than the outer diameter of the catheter.

Preferably, the sealing belt is connected to the balloon main body.

Preferably, the balloon is packaged in a capsule before being swallowed, one end of the catheter is connected to the self-sealing valve on the balloon while the other end is left outside the capsule, and the catheter has a certain length, making the in-vitro end leave outside the oral cavity during use.

Preferably, the balloon includes an upper-layer main body and a lower-layer main body, and the self-sealing valve includes an upper-layer self-sealing valve and a lower-layer self-sealing valve.

Preferably, a length of the pocket membrane is not greater than that of the upper-layer self-sealing valve.

Preferably, the sealing belt includes a left-side sealing strip and a right-side sealing strip, which are on both sides of the distal end of the upper-layer self-sealing valve or the lower-layer self-sealing valve, respectively.

Preferably, the left-side sealing strip and the right-side sealing strip are a whole with the upper-layer self-sealing valve or the lower-layer self-sealing valve, or connected together through different materials in a bonding manner or in a welding manner.

Preferably, the pocket membrane is connected to the lower-layer self-sealing valve along the connecting belt in a bonding manner or in a welding manner, in order to form the inverted pocket, and the small hole is formed in the inverted pocket.

Preferably, while the upper-layer main body and the lower-layer main body are combined together at the edge in a bonding manner or in a welding manner, three edges of the upper-layer self-sealing valve are connected to the lower-layer self-sealing valve and the inverted pocket through the connecting belt, in order to form the self-sealing valve.

Preferably, the catheter is threaded in from unconnected ends of the upper-layer self-sealing valve and the lower-layer self-sealing valve and threaded out from the small hole, to enter any position of the inverted pocket.

Preferably, the left-side sealing strip and the right-side sealing strip are adhered to the balloon, respectively.

Preferably, overturn is performed through the hole in the upper-layer main body after the combination is completed, the upper-layer main body and the lower-layer main body form the balloon, the self-sealing valve enters the inside of the balloon together with the inverted pocket and the sealing belt that are at the distal end of the self-sealing valve, one end of the catheter is outside the balloon while the other end is inside the inverted pocket, and finally the hole in the upper-layer main body is sealed with a patch.

The present disclosure provides a method for making a self-sealing system in claims, a balloon main body includes an upper-layer main body and a lower-layer main body, a self-sealing valve includes an upper-layer self-sealing valve and a lower-layer self-sealing valve, where while the upper-layer main body and the lower-layer main body are combined together at the edge in a bonding manner or in a welding manner, three edges of the upper-layer self-sealing valve and the lower-layer self-sealing valves are adhered or welded together through a connecting belt, in order to form the self-sealing valve; a small hole is formed in the lower-layer self-sealing valve, all or a part of the lower-layer self-sealing valve and an upper-layer inverted pocket membrane are adhered or welded together with a lower-layer inverted pocket membrane through the connecting belt, in order to form an inverted pocket; and a catheter is threaded in from unconnected ends of the upper-layer self-sealing valve and the lower-layer self-sealing valve and threaded out from the small hole, to enter any position of the inverted pocket.

Preferably, the sealing belt is outside the inverted pocket and includes an upper-layer sealing strip and a lower-layer sealing strip, where the upper-layer sealing strip is at the outside of the upper-layer inverted pocket membrane, the lower-layer sealing strip is at the outside of the lower-layer inverted pocket membrane, and the sealing belt is adhered to the balloon through both ends of the upper-layer sealing strip.

Preferably, overturn is performed through the hole in the upper-layer main body after the combination is completed, the upper-layer main body and the lower-layer main body form the balloon main body, the self-sealing valve enters the inside of the balloon main body together with the inverted pocket and the sealing belt that are at the distal end of the self-sealing valve, one end of the catheter is outside the balloon while the other end is inside the inverted pocket, and finally the hole in the upper-layer main body is sealed with a patch.

The present disclosure provides another method for making a self-sealing system, a balloon main body includes an upper-layer main body and a lower-layer main body, a self-sealing valve includes an upper-layer self-sealing valve and a lower-layer self-sealing valve, where while the upper-layer main body and the lower-layer main body are combined together at the edge in a bonding manner or in a welding manner, three edges of the upper-layer self-sealing valve and the lower-layer self-sealing valves are adhered or welded together through a connecting belt, in order to form the self-sealing valve; a small hole is formed in the lower-layer self-sealing valve, all or a part of the lower-layer self-sealing valve and an upper-layer inverted pocket membrane are adhered or welded together with a lower-layer inverted pocket membrane through the connecting belt, in order to form an inverted pocket; and a catheter is threaded in from unconnected ends of the upper-layer self-sealing valve and the lower-layer self-sealing valve and threaded out from the small hole, to enter any position of the inverted pocket.

Preferably, the sealing belt is outside the inverted pocket and includes an upper-layer sealing strip and a lower-layer sealing strip, where the upper-layer sealing strip is at the outside of the upper-layer inverted pocket membrane, the lower-layer sealing strip is at the outside of the lower-layer inverted pocket membrane, and the sealing belt is adhered to the balloon through both ends of the lower-layer sealing strip.

Preferably, overturn is performed through the hole in the upper-layer main body after the combination is completed, the upper-layer main body and the lower-layer main body form the balloon main body, the self-sealing valve enters the inside of the balloon main body together with the inverted pocket and the sealing belt that are at the distal end of the self-sealing valve, one end of the catheter is outside the balloon while the other end is inside the inverted pocket, and finally the hole in the upper-layer main body is sealed with a patch.

The present disclosure further provides a method for making a self-sealing system, a balloon includes an upper-layer main body and a lower-layer main body, a self-sealing valve includes an upper-layer self-sealing valve and a lower-layer self-sealing valve, where a small hole is formed in a pocket membrane for making an inverted pocket, a perimeter of the small hole is not greater than an outer diameter of a catheter, a length of the pocket membrane is not greater than that of the upper-layer self-sealing valve, and a sealing belt includes a left-side sealing strip and a right-side sealing strip, which are on both sides of a distal end of the upper-layer self-sealing valve or the lower-layer self-sealing valve, respectively.

Preferably, the pocket membrane is connected to the lower-layer self-sealing valve along the connecting belt in a bonding manner or in a welding manner, in order to form the inverted pocket, and the small hole is formed in the inverted pocket. While the upper-layer main body and the lower-layer main body are combined together at the edge in a bonding manner or in a welding manner, three edges of the upper-layer self-sealing valve are connected to the lower-layer self-sealing valve and the inverted pocket through the connecting belt, in order to form the self-sealing valve; a catheter is threaded in from unconnected ends of the upper-layer self-sealing valve and the lower-layer self-sealing valve and threaded out from the small hole, to enter any position of the inverted pocket; and the left-side sealing strip and the right-side sealing strip are adhered to the balloon, respectively.

Preferably, overturn is performed through the hole in the upper-layer main body after the combination is completed, the upper-layer main body and the lower-layer main body form the balloon, the self-sealing valve enters the inside of the balloon together with the inverted pocket and the sealing belt that are at the distal end of the self-sealing valve, one end of the catheter is outside the balloon while the other end is inside the inverted pocket, and finally the hole in the upper-layer main body is sealed with a patch.

The self-sealing system in this solution is not only used for an intragastric balloon system, but also used for other balloon implants, such as a skin inflatable balloon.

According to the self-sealing system in The present disclosure, the sealing belt may make the distal end of the inverted pocket be tightly closed after the filling is full and the catheter is removed, in order to prevent the filling from leaking out.

### BRIEF DESCRIPTION OF THE DRAWINGS

FIG. 1 is a diagram of a self-sealing valve with a catheter and a balloon according to the prior art;
FIG. 2 is a connecting diagram of a balloon and a catheter according to one implementation mode of The present disclosure;
FIG. 3 is a breakdown diagram of a balloon main body and a valve according to one implementation mode of The present disclosure;
FIG. 4 is a breakdown diagram of a balloon main body, a valve and an inverted pocket according to one implementation mode of The present disclosure;
FIG. 5 is a diagram of a valve, a pocket and a catheter according to one implementation mode of The present disclosure;
FIG. 6 is a diagram of a valve, a pocket, a catheter and a sealing belt according to one implementation mode of The present disclosure;
FIG. 7 is a diagram of a self-sealing system according to one implementation mode of The present disclosure;
FIG. 8 is a balloon, a self-sealing valve, an inverted pocket, a sealing belt and a breakdown diagram thereof according to another implementation mode of The present disclosure; and
FIG. 9 is a catheter, a self-sealing valve, an inverted pocket, a sealing belt and an assembly diagram thereof according to another implementation mode of The present disclosure.

### DETAILED DESCRIPTION OF THE EMBODIMENTS

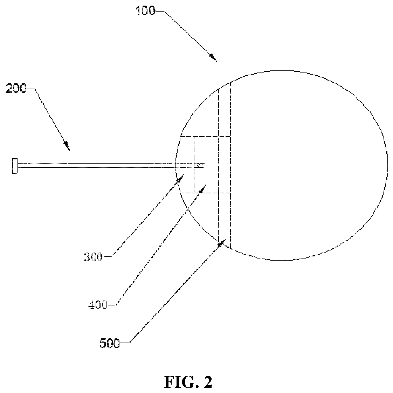

Referring to FIG. 2, which illustrates a structural diagram of an intragastric balloon system according to one implementation mode of The present disclosure. The intragastric balloon system includes a balloon 100, a catheter 200, a capsule and a filling (not shown in the figure), and the balloon 100 includes a self-sealing valve 300, an inverted pocket 400 and a sealing belt 500. The balloon 100 is packaged in the capsule before being swallowed, one end (that is, an in-vivo end) of the catheter 200 is connected to the self-sealing valve 300 on the balloon 100 while the other end (that is, an in-vitro end) is left outside the capsule, and the catheter 200 has a certain length, making the in-vitro end leave outside an oral cavity during use.

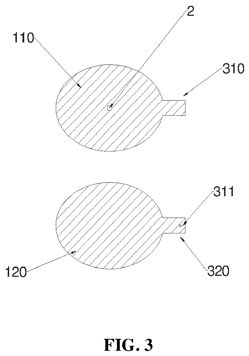

Referring to FIG. 3, which illustrates a balloon 100, a self-sealing valve 300 and a breakdown diagram thereof; the balloon 100 includes an upper-layer main body 110 and a lower-layer main body 120, and the self-sealing valve 300 includes an upper-layer self-sealing valve 310 and a lower-layer self-sealing valve 320; and a small hole 311 is formed in the lower-layer self-sealing valve 320, and a perimeter of the small hole 311 is not greater than an outer diameter of the catheter 200. The upper-layer seal-sealing valve 310 and the upper-layer main body 110 may be a whole, or may also be connected together through different materials in a bonding manner or in a welding manner. Similarly, the lower-layer seal-sealing valve 320 and the lower-layer main body 120 may be a whole, or may also be connected together through different materials in a bonding manner or in a welding manner.

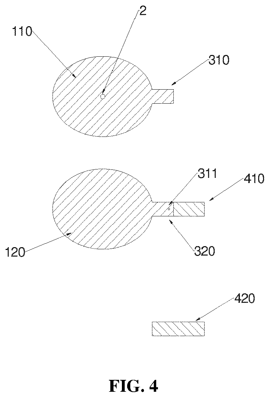

Referring to FIG. 4, which illustrates a balloon 100, a self-sealing valve 300, an inverted pocket 400 and a breakdown diagram thereof. The inverted pocket 400 includes an upper-layer inverted pocket membrane 410 and a lower-layer inverted pocket membrane 420, and the upper-layer inverted pocket membrane 410 may be an extension of the lower-layer self-sealing valve 320, or the upper-layer inverted pocket membrane 410 and the lower-layer self-sealing valve 320 may also be connected together through different materials in a bonding manner or in a welding manner. A length of the lower-layer inverted pocket membrane 420 is greater than that of the upper-layer inverted pocket membrane 410.

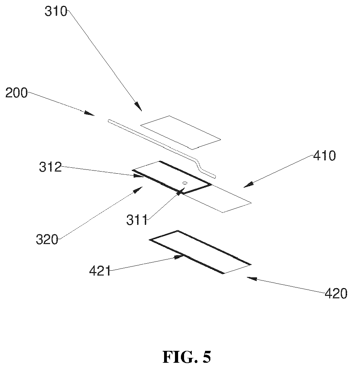

Referring to FIG. 5, which illustrates a catheter 200, a self-sealing valve 300, an inverted pocket 400 and a breakdown diagram thereof. While the upper-layer main body 110 and the lower-layer main body 120 are combined together at the edge in a bonding manner or in a welding manner, three edges of the upper-layer self-sealing valve 310 and the lower-layer self-sealing valve 320 are bonded or welded together through a connecting belt 312, in order to form the self-sealing valve 300. All or a part of the lower-layer self-sealing valve 320 and the upper-layer inverted pocket membrane 410 are bonded or welded with the lower-layer inverted pocket membrane 420 through the connecting belt 421, in order to form the inverted pocket 400. The catheter 200 is threaded in from unconnected ends of the upper-layer self-sealing valve 310 and the lower-layer self-sealing valve 320 and threaded out from the small hole 311, to enter any position of the inverted pocket 400.

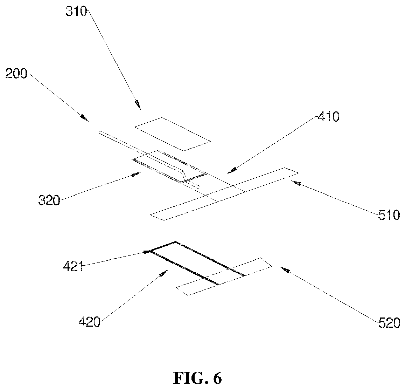

Referring to FIG. 6, which illustrates a catheter 200, a self-sealing valve 300, an inverted pocket 400, a sealing belt 500 and a breakdown diagram thereof. The sealing belt 500 is outside the inverted pocket 400 and includes an upper-layer sealing strip 510 and a lower-layer sealing strip 520, where the upper-layer sealing strip 510 is at the outside of the upper-layer inverted pocket membrane 410, and the lower-layer sealing strip 520 is at the outside of the lower-layer inverted pocket membrane 420. A length of the upper-layer sealing strip 510 is not less than that of the lower-layer sealing strip 520, and a length of the lower-layer sealing strip 520 is greater than a width of the lower-layer inverted pocket membrane 420. Two sides of the lower-layer sealing strip 520 are directly or indirectly connected to the upper-layer sealing strip 510 through the inverted pocket 400 in a bonding manner or a welding manner, in order to form the sealing belt 500.

The sealing belt 500 is adhered to the balloon 100 through two ends of the upper-layer sealing strip 510.

Certainly, those skilled in the art can also understand that, alternatively, a length of the lower-layer sealing strip 520 is not less than that of the upper-layer sealing strip 510, and a length of the upper-layer sealing strip 510 is greater than a width of the upper-layer inverted pocket membrane 410. Two sides of the upper-layer sealing strip 510 are directly or indirectly connected to the lower-layer sealing strip 520 through the inverted pocket 400 in a bonding manner or a welding manner, in order to form the sealing belt. The sealing belt 500 is adhered to the balloon through two ends of the lower-layer sealing strip 520.

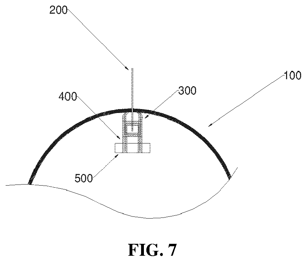

Referring to FIG. 7, overturn is performed through the hole 2 in the upper-layer main body 110 after the combination is completed, the upper-layer main body 110 and the lower-layer main body 120 form the balloon 100, and the self-sealing valve 300 enters the inside of the balloon 100 together with the inverted pocket 400 and the sealing belt 500 that are at the distal end of the self-sealing valve 300. One end of the catheter 200 is outside the balloon 100 while the other end is inside the inverted pocket 400. Finally, the hole 2 in the upper-layer main body 110 is sealed with a patch.

The use situation of the intragastric balloon according to this embodiment is as follows: after a patient swallows the capsule including the balloon 100 and a part of the catheter 200, the capsule dissolves rapidly in an intragastric environment, the filling is conveyed to the balloon 100 from the in-vitro end of the catheter 200, and the balloon 100 becomes an expanded state from an initial compressed state. Since the diameter of the small hole 311 is not greater than the outer diameter of the catheter 200, the catheter 200 is in gapless close fit with the small hole 311, to ensure the whole tightness of the balloon 100 and the self-sealing valve when conveying the filling, and the filling will not leak out. The catheter 200 is removed after the filling is completed, the sealing belt 500 tightens the distal end of the inverted pocket 400 such that the upper-layer inverted pocket membrane 410 fits with the lower-layer inverted pocket membrane 420 closely, to block the leakage of the filling in the balloon 100. The balloon 100 occupies the volume of the stomach for a long time, thereby achieving the weight-loss purpose.

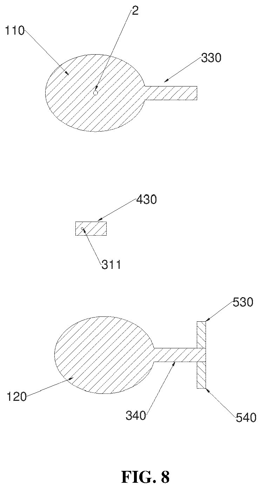

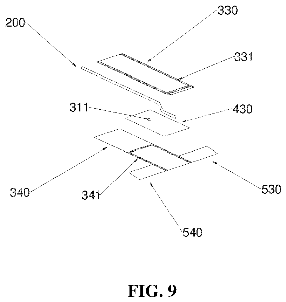

FIG. 8 and FIG. 9 illustrate a structural diagram of an intragastric balloon system according to another implementation mode of The present disclosure.

Referring to FIG. 8, which illustrates a balloon 100, a self-sealing valve 300, an inverted pocket 400, a sealing belt 500 and a breakdown diagram thereof. The balloon 100 includes an upper-layer main body 110 and a lower-layer main body 120, the self-sealing valve 300 includes an upper-layer seal-sealing valve 330 and a lower-layer self-sealing valve 340, the upper-layer seal-sealing valve 330 and the upper-layer main body 110 may be a whole, or may also be connected together through different materials in a bonding manner or in a welding manner. Similarly, the lower-layer seal-sealing valve 340 and the lower-layer main body 120 may be a whole, or may also be connected together through different materials in a bonding manner or in a welding manner. A small hole 311 is formed in a pocket membrane 430 for making the inverted pocket 400, and a perimeter of the small hole 311 is not greater than an outer diameter of a catheter 200. A length of the pocket membrane 430 is not greater than that of the upper-layer self-sealing valve 330. The sealing belt 500 includes a left-side sealing strip 530 and a right-side sealing strip 540, which are on both sides of a distal end of the lower-layer self-sealing valve 340; and the left-side sealing strip 530 and the right-side sealing strip 540 may be a whole with the lower-layer self-sealing valve 340, or connected together through different materials in a bonding manner or in a welding manner.

Referring to FIG. 9, which illustrates a catheter 200, a self-sealing valve 300, an inverted pocket 400, a sealing belt 500 and an assembly diagram thereof. The pocket membrane 430 is connected to the lower-layer self-sealing valve 340 along a connecting belt 341 in a bonding manner or in a welding manner, in order to form the inverted pocket 400, and a small hole 311 is formed in the inverted pocket. While an upper-layer main body 110 and a lower-layer main body 120 are combined together at an edge in a bonding manner or in a welding manner, three edges of an upper-layer self-sealing valve 330 are connected to a lower-layer self-sealing valve 340 and the inverted pocket 400 through a connecting belt 331, in order to form the self-sealing valve 300. The catheter 200 is threaded in from unconnected ends of the upper-layer self-sealing valve 330 and the lower-layer self-sealing valve 340 and threaded out from the small hole 311, to enter any position of the inverted pocket 400.

A left-side sealing strip 530 and a right-side sealing strip 540 of the sealing belt 500 are adhered to the balloon 100, respectively.

Referring to FIG. 7, overturn is performed through a hole 2 in the upper-layer main body 110 after the combination is completed, the upper-layer main body 110 and the lower-layer main body 120 form the balloon 100, and the self-sealing valve 300 enters the inside of the balloon 100 together with the inverted pocket 400 and the sealing belt 500 that are at the distal end of the self-sealing valve 300. One end of the catheter 200 is outside the balloon 100 while the other end is inside the inverted pocket. Finally, the hole 2 in the upper-layer main body 110 is sealed with a patch.

The use situation of the intragastric balloon according to this embodiment is as follows: after a patient swallows the capsule including the balloon 100 and a part of the catheter 200, the capsule dissolves rapidly in an intragastric environment, the filling is conveyed to the balloon 100 from the in-vitro end of the catheter 200, and the balloon 100 becomes an expanded state from an initial compressed state. Since the diameter of the small hole 311 is not greater than the outer diameter of the catheter 200, the catheter 200 is in gapless close fit with the small hole 311, to ensure the whole tightness of the balloon 100 and the self-sealing valve when conveying the filling, and the filling will not leak out. The catheter 200 is removed after the filling is completed, the sealing belt 500 tightens the distal end of the inverted pocket 400 such that the upper-layer inverted pocket membrane 410 fits with the lower-layer inverted pocket membrane 420 closely, to block the leakage of the filling in the balloon 100. The balloon 100 occupies the volume of the stomach for a long time, thereby achieving the weight-loss purpose.

The above disclosed is only several preferred embodiments of The present disclosure, instead of limiting the claim protection scope of the present disclosure, therefore any equal change made based on the scope of the patent applied in The present disclosure still belongs to
the scope covered by The present disclosure.

## Claims

1. A self-sealing system of a balloon system implanted in vivo, wherein the balloon system comprises a balloon, a catheter and a filling; the balloon comprises a self-sealing valve, which penetrates through the catheter and is hermetically connected to the catheter; and a distal end of the self-sealing valve is provided with an inverted pocket and a sealing belt.

2. The self-sealing system according to claim 1, wherein the catheter penetrates through a small hole on the self-sealing valve and enters the inverted pocket.

3. The self-sealing system according to claim 2, wherein a sealing belt is connected to a balloon.

4. The self-sealing system according to claim 3, wherein the balloon is packaged in a capsule before being swallowed, one end of a catheter is connected to a self-sealing valve on the balloon while the other end is left outside the capsule, and the catheter has a certain length, making an in-vitro end leave outside an oral cavity during use.

5. The self-sealing system according to claim 4, wherein the balloon comprises an upper-layer main body and a lower-layer main body, and the self-sealing valve comprises an upper-layer self-sealing valve and a lower-layer self-sealing valve.

6. The self-sealing system according to claim 5, wherein a small hole is formed in the lower-layer self-sealing valve, and a perimeter of the small hole is not greater than an outer diameter of a catheter.

7. The self-sealing system according to claim 6, wherein an upper-layer seal-sealing valve and an upper-layer main body are a whole, or connected together through different materials in a bonding manner or in a welding manner.

8. The self-sealing system according to claim 6, wherein the lower-layer seal-sealing valve and a lower-layer main body are a whole, or connected together through different materials in a bonding manner or in a welding manner.

9. The self-sealing system according to any one of claims 7 to 8, wherein an inverted pocket comprises an upper-layer inverted pocket membrane and a lower-layer inverted pocket membrane.

10. The self-sealing system according to claim 9, wherein the upper-layer inverted pocket membrane is an extension of a lower-layer self-sealing valve, or the upper-layer inverted pocket membrane and the lower-layer self-sealing valve are connected together through different materials in a bonding manner or in a welding manner.

11. The self-sealing system according to claim 9, wherein a length of the lower-layer inverted pocket membrane is greater than that of the upper-layer inverted pocket membrane.

12. The self-sealing system according to claim 11, wherein while an upper-layer main body and a lower-layer main body are combined together at an edge in a bonding manner or in a welding manner, three edges of an upper-layer self-sealing valve and a lower-layer self-sealing valve are bonded or welded together through a connecting belt, in order to form a self-sealing valve.

13. The self-sealing system according to claim 12, wherein all or a part of the lower-layer self-sealing valve and an upper-layer inverted pocket membrane are bonded or welded with a lower-layer inverted pocket membrane through the connecting belt, in order to form an inverted pocket; and a catheter is threaded in from unconnected ends of the upper-layer self-sealing valve and the lower-layer self-sealing valve and threaded out from a small hole, to enter any position of the inverted pocket.

14. The self-sealing system according to claim 13, wherein a sealing belt is outside the inverted pocket.

15. The self-sealing system according to claim 14, wherein the sealing belt comprises an upper-layer sealing strip and a lower-layer sealing strip, the upper-layer sealing strip is at the outside of an upper-layer inverted pocket membrane, and the lower-layer sealing strip is at the outside of a lower-layer inverted pocket membrane.

16. The self-sealing system according to claim 15, wherein a length of the upper-layer sealing strip is not less than that of the lower-layer sealing strip, and a length of the lower-layer sealing strip is greater than a width of the lower-layer inverted pocket membrane.

17. The self-sealing system according to claim 16, wherein two sides of the lower-layer sealing strip are directly or indirectly connected to the upper-layer sealing strip through an inverted pocket in a bonding manner or a welding manner, in order to form a sealing belt.

18. The self-sealing system according to claim 17, wherein the sealing belt is adhered to a balloon through two ends of the upper-layer sealing strip.

19. The self-sealing system according to claim 15, wherein a length of the lower-layer sealing strip is not less than that of the upper-layer sealing strip, and a length of the upper-layer sealing strip is greater than a width of the upper-layer inverted pocket membrane.

20. The self-sealing system according to claim 19, wherein two sides of the lower-layer sealing strip are directly or indirectly connected to the lower-layer sealing strip through an inverted pocket in a bonding manner or a welding manner, in order to form a sealing belt.

21. The self-sealing system according to claim 20, wherein the sealing belt is adhered to a balloon through two ends of the lower-layer sealing strip.

22. The self-sealing system according to claim 18 or 21, wherein overturn is performed through a hole in an upper-layer main body after the combination is completed, the upper-layer main body and a lower-layer main body form the balloon, a self-sealing valve enters the inside of the balloon together with an inverted pocket and the sealing belt that are at a distal end of the self-sealing valve, one end of a catheter is outside the balloon while the other end is inside the inverted pocket, and finally the hole in the upper-layer main body is sealed with a patch.

23. The self-sealing system according to claim 1, wherein a small hole is formed in a pocket membrane of the inverted pocket, and a perimeter of the small hole is not greater than an outer diameter of the catheter.

24. The self-sealing system according to claim 23, wherein a sealing belt is connected to a balloon.

25. The self-sealing system according to claim 24, wherein the balloon is packaged in a capsule before being swallowed, one end of a catheter is connected to a self-sealing valve on the balloon while the other end is left outside the capsule, and the catheter has a certain length, making an in-vitro end leave outside an oral cavity during use.

26. The self-sealing system according to claim 25, wherein the balloon comprises an upper-layer main body and a lower-layer main body, and the self-sealing valve comprises an upper-layer self-sealing valve and a lower-layer self-sealing valve.

27. The self-sealing system according to claim 26, wherein a length of a pocket membrane is not greater than that of the upper-layer self-sealing valve.

28. The self-sealing system according to claim 27, wherein a sealing belt comprises a left-side sealing strip and a right-side sealing strip, which are on both sides of a distal end of the upper-layer self-sealing valve or a lower-layer self-sealing valve, respectively.

29. The self-sealing system according to claim 28, wherein the left-side sealing strip and the right-side sealing strip are a whole with the upper-layer self-sealing valve or the lower-layer self-sealing valve, or connected together through different materials in a bonding manner or in a welding manner.

30. The self-sealing system according to claim 29, wherein a pocket membrane is connected to the lower-layer self-sealing valve along a connecting belt in a bonding manner or in a welding manner, in order to form an inverted pocket, and a small hole is formed in the inverted pocket.

31. The self-sealing system according to claim 30, wherein while an upper-layer main body and a lower-layer main body are combined together at an edge in a bonding manner or in a welding manner, three edges of an upper-layer self-sealing valve are connected to the lower-layer self-sealing valve and the inverted pocket through the connecting belt, in order to form a self-sealing valve.

32. The self-sealing system according to claim 31, wherein a catheter is threaded in from unconnected ends of the upper-layer self-sealing valve and the lower-layer self-sealing valve and threaded out from a small hole, to enter any position of the inverted pocket.

33. The self-sealing system according to claim 32, wherein a left-side sealing strip and a right-side sealing strip are adhered to a balloon, respectively.

34. The self-sealing system according to claim 33, wherein overturn is performed through a hole in an upper-layer main body after the combination is completed, the upper-layer main body and a lower-layer main body form the balloon, a self-sealing valve enters the inside of the balloon together with an inverted pocket and a sealing belt that are at a distal end of the self-sealing valve, one end of a catheter is outside the balloon while the other end is inside the inverted pocket, and finally the hole in the upper-layer main body is sealed with a patch.

35. A method for making a self-sealing system in any one of claims 1 to 18 and 22, the balloon main body comprises the upper-layer main body and the lower-layer main body, a self-sealing valve comprises the upper-layer self-sealing valve and the lower-layer self-sealing valve, wherein while the upper-layer main body and the lower-layer main body are combined together at the edge in a bonding manner or in a welding manner, three edges of the upper-layer self-sealing valve and the lower-layer self-sealing valves are adhered or welded together through the connecting belt, in order to form the self-sealing valve; the small hole is formed in the lower-layer self-sealing valve, all or a part of the lower-layer self-sealing valve and the upper-layer inverted pocket membrane are adhered or welded together with the lower-layer inverted pocket membrane through the connecting belt, in order to form the inverted pocket; and the catheter is threaded in from the unconnected ends of the upper-layer self-sealing valve and the lower-layer self-sealing valve and threaded out from the small hole, to enter any position of the inverted pocket.

36. The method according to claim 35, wherein a sealing belt is outside the inverted pocket and comprises an upper-layer sealing strip and a lower-layer sealing strip, the upper-layer sealing strip is at the outside of the upper-layer inverted pocket membrane, the lower-layer sealing strip is at the outside of the lower-layer inverted pocket membrane, and the sealing belt is adhered to the balloon through both ends of the upper-layer sealing strip.

37. The method according to claim 36, wherein overturn is performed through a hole in an upper-layer main body after the combination is completed, the upper-layer main body and a lower-layer main body form a balloon main body, a self-sealing valve enters the inside of the balloon main body together with the inverted pocket and the sealing belt that are at a distal end of the self-sealing valve, one end of a catheter is outside the balloon while the other end is inside the inverted pocket, and finally the hole in the upper-layer main body is sealed with a patch.

38. A method for making a self-sealing system in any one of claims 1 to 15 and 19 to 22, the balloon main body comprises the upper-layer main body and the lower-layer main body, the self-sealing valve comprises the upper-layer self-sealing valve and the lower-layer self-sealing valve, wherein while the upper-layer main body and the lower-layer main body are combined together at the edge in a bonding manner or in a welding manner, three edges of the upper-layer self-sealing valve and the lower-layer self-sealing valves are adhered or welded together through the connecting belt, in order to form the self-sealing valve; the small hole is formed in the lower-layer self-sealing valve, all or a part of the lower-layer self-sealing valve and the upper-layer inverted pocket membrane are adhered or welded together with the lower-layer inverted pocket membrane through the connecting belt, in order to form the inverted pocket; and the catheter is threaded in from the unconnected ends of the upper-layer self-sealing valve and the lower-layer self-sealing valve and threaded out from the small hole, to enter any position of the inverted pocket.

39. The method according to claim 38, wherein a sealing belt is outside the inverted pocket and comprises an upper-layer sealing strip and a lower-layer sealing strip, the upper-layer sealing strip is at the outside of the upper-layer inverted pocket membrane, the lower-layer sealing strip is at the outside of the lower-layer inverted pocket membrane, and the sealing belt is adhered to a balloon through both ends of the upper-layer sealing strip.

40. The method according to claim 39, wherein overturn is performed through a hole in an upper-layer main body after the combination is completed, the upper-layer main body and a lower-layer main body form a balloon main body, a self-sealing valve enters the inside of the balloon main body together with the inverted pocket and the sealing belt that are at a distal end of the self-sealing valve, one end of a catheter is outside the balloon while the other end is inside the inverted pocket, and finally the hole in the upper-layer main body is sealed with a patch.

41. A method for making a self-sealing system in any one of claims 1 and 23 to 34, the balloon comprises the upper-layer main body and the lower-layer main body, a self-sealing valve comprises an upper-layer self-sealing valve and a lower-layer self-sealing valve, wherein the small hole is formed in the pocket membrane for making the inverted pocket, the perimeter of the small hole is not greater than the outer diameter of the catheter, the length of the pocket membrane is not greater than that of the upper-layer self-sealing valve, and the sealing belt comprises the left-side sealing strip and the right-side sealing strip, which are on both sides of the distal end of the upper-layer self-sealing valve or the lower-layer self-sealing valve, respectively.

42. The method according to claim 41, wherein the pocket membrane is connected to the lower-layer self-sealing valve along a connecting belt in a bonding manner or in a welding manner, in order to form the inverted pocket, and the small hole is formed in the inverted pocket; and while the upper-layer main body and the lower-layer main body are combined together at the edge in a bonding manner or in a welding manner, three edges of the upper-layer self-sealing valve are connected to the lower-layer self-sealing valve and the inverted pocket through a connecting belt, in order to form the self-sealing valve; the catheter is threaded in from unconnected ends of the upper-layer self-sealing valve and the lower-layer self-sealing valve and threaded out from the small hole, to enter any position of the inverted pocket; and the left-side sealing strip and the right-side sealing strip are adhered to the balloon, respectively.

43. The method according to claim 42, wherein overturn is performed through the hole in the upper-layer main body after the combination is completed, the upper-layer main body and the lower-layer main body form the balloon, the self-sealing valve enters the inside of the balloon together with the inverted pocket and the sealing belt that are at the distal end of the self-sealing valve, one end of the catheter is outside the balloon while the other end is inside the inverted pocket, and finally the hole in the upper-layer main body is sealed with a patch.

44. A balloon system, comprising a self-sealing system according to any one of claims 1 to 43.

45. The balloon system according to claim 44, wherein the balloon system is an intragastric balloon system.

46. The balloon system according to claim 45, wherein the balloon system is a skin inflatable balloon system.
